# EUROPEAN PATENT APPLICATION

(11) **EP 4 492 914 A1**
(43) Date of publication of application: **15.01.2025**
(21) Application number: 23189988.1
(22) Date of filing: 07.08.2023
(51) Int. Cl.: H05B 3/34, F21V 29/90, F21L 4/00

(54) **A LIGHT WITH A HEATING FUNCTION**

(30) Priority: 14.07.2023 CN 202321857241 U
(71) Applicant: Ningbo Bright Electric Co.,Ltd, 315000 Ningbo Zhejiang (CN)
(72) Inventor: HU, ZHENFEI, NINGBO, 315000 (CN)
(74) Representative: Zaboliene, Reda

(57) **Abstract**

The present utility model relates to a light with a heating function, including a PCB, a light cylinder, a heating sheet, and a sleeve, wherein the heating sheet is electrically connected to the PCB, the sleeve is sheathed outside the light cylinder, and the heating sheet is disposed between the light cylinder and the sleeve; and including a heating switch and a power supply, wherein the power supply and the heating switch are electrically connected to the PCB separately, and the power supply and the PCB are mounted inside the light cylinder. The objective of the present utility model is to provide a light with a heating function, in order to solve a problem that hands are prone to frostbite when portable lights in the prior art are used in cold conditions.

## Description

### Reference To Prior Application

This application claims priority to Chinese Patent Application 202321857241.9, filed on July 14, 2023.

### Technical Field

The present utility model relates to the technical field of lights, in particular to a light with a heating function.

### Background

With the development of economy, the styles and types of lights are increasing. People who often travel or go on a business trip sometimes carry a light, such as a flashlight. When the flashlight is used in cold conditions, hands exposed to the outside are prone to frostbite.

### Summary

The objective of the present utility model is to provide a light with a heating function, in order to solve a problem that hands are prone to frostbite when portable lights in the prior art are used in cold conditions.

In order to achieve the above objective, a basic solution of the present utility model is as follows:
A light with a heating function includes a printed circuit board (PCB), a light cylinder, a heating sheet, and a sleeve, wherein the heating sheet is electrically connected to the PCB, the sleeve is sheathed outside the light cylinder, and the heating sheet is disposed between the light cylinder and the sleeve; and includes a heating switch and a power supply, wherein the power supply and the heating switch are electrically connected to the PCB separately, and the power supply and the PCB are mounted inside the light cylinder.

Compared with the prior art, the light with a heating function in the present application has the following beneficial effects:
The light in the present application is equipped with a power supply, a PCB, a heating sheet, and a sleeve, wherein the heating sheet is supplied with power to generate heat, and the heat is transferred to the outside of the light through the sleeve, so that the user does not get frostbitten in cold conditions.

Preferably, a gasket is disposed between the light cylinder and the heating sheet.

Beneficial effect: the gasket is configured to isolate the heat generated by the heating sheet, so as to prevent the heat from entering the interior of the light cylinder to affect normal discharge of the power supply.

Preferably, a heating seat is disposed on an outer surface of the light cylinder, and the heating seat is adapted to the gasket.

Beneficial effect: the heating seat can prevent displacement of the gasket to some extent, meanwhile the heating seat facilitates assembly of the gasket.

Preferably, the gasket is made of ethylene-propylene-diene monomer (EPDM) rubber.

Beneficial effect: the gasket made of EPDM rubber has strong heat resistance and can be used for a long term in high-temperature environments.

Preferably, the sleeve is made of aluminum.

Beneficial effect: the aluminum has good thermal conductivity, which can improve thermal conductivity efficiency of the heating sheet inside the light.

Preferably, the PCB is electrically connected with a thermistor, which is placed on the gasket.

Beneficial effect: the thermistor is used for controlling the temperature of the heating sheet to prevent excessive heat generated by the heating sheet from scalding hands.

### Brief Description of the Drawings

FIG. 1 is a schematic structural diagram of a light with a heating function according to an embodiment of the present utility model;
FIG. 2 is an exploded schematic diagram of a light cylinder according to an embodiment of the present utility model;
FIG. 3 is a schematic structural diagram of a power supply, a PCB, and a heating switch according to an embodiment of the present utility model; and
FIG. 4 is a schematic structural diagram of a heating sheet, a light cylinder, and a gasket according to an embodiment of the present utility model.

### Detailed Description of the Embodiments

The following is a further detailed explanation through a specific embodiment.

Reference numerals in the drawings include: a PCB 1, a light cylinder 2, a heating sheet 3, a sleeve 4, a heating switch 5, a power supply 6, a gasket 7, and a heating seat 8.

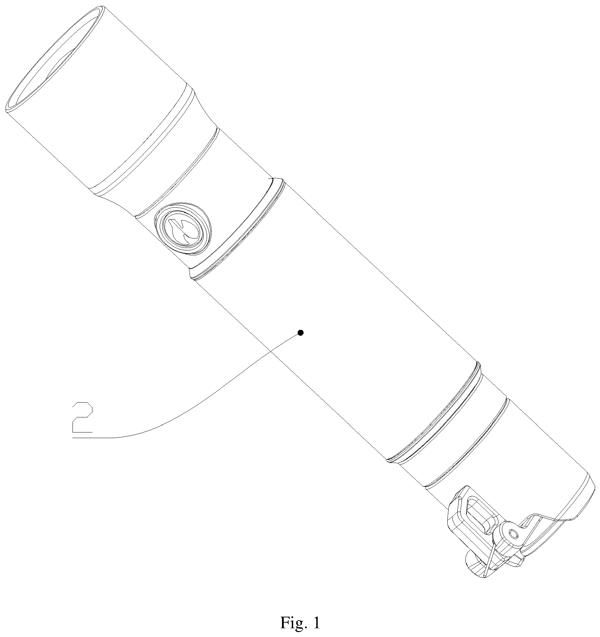

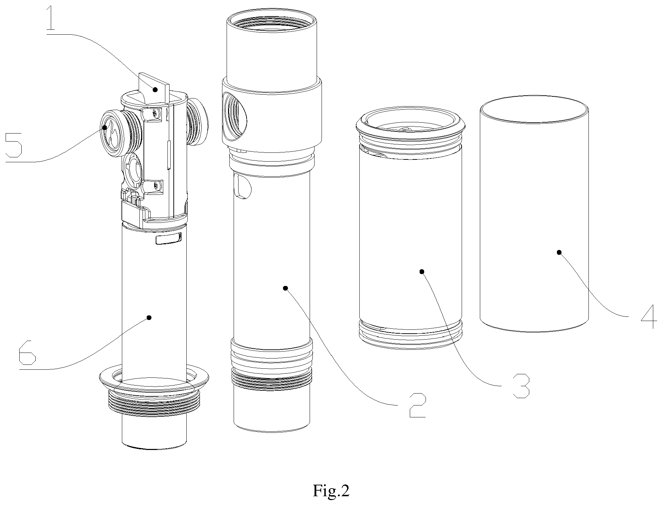

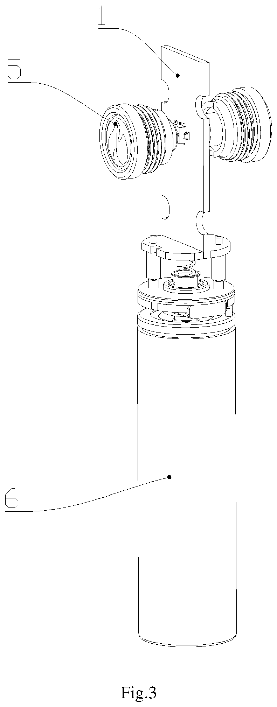

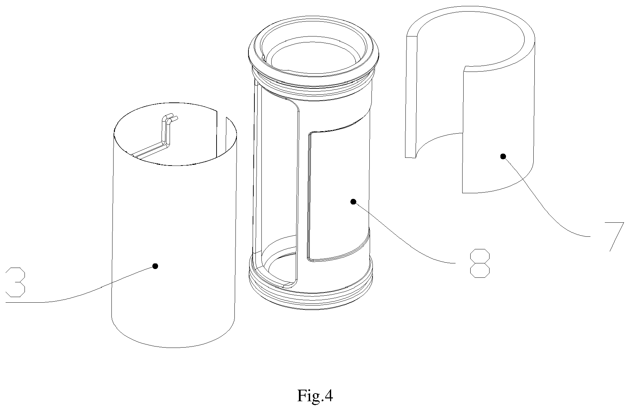

As shown in FIGs. 1-4, this embodiment shows a light with a heating function. A light body is disposed at a top of the light, and a middle part and bottom of the light form the light cylinder 2.

The light includes a PCB 1, a heating sheet 3, and a sleeve 4. The heating sheet 3 is electrically connected to the PCB 1, the sleeve 4 is sheathed outside the light cylinder 2, and the heating sheet 3 is disposed between the light cylinder 2 and the sleeve 4. The light further includes a heating switch 5 and a power supply 6, the power supply 6 and the heating switch 5 are electrically connected to the PCB 1 separately, and the power supply 6 and the PCB 1 are mounted inside the light cylinder 2.

Specifically, the heating switch 5 is mounted on one side of the PCB 1, and the power supply 6 is mounted at a bottom of the PCB 1. The power supply 6 and the PCB 1 are mounted inside the light cylinder 2, and through holes through which the heating switch 5 passes are disposed on two sides of a top of the light cylinder 2.

When a user uses the light in this embodiment, the heating sheet 3 may be activated through the heating switch 5 to generate heat. After the heating sheet 3 generates heat, the heat is transferred to user's hands through the sleeve 4 to prevent the hands from being frostbitten when the user uses the light in cold conditions.

In some embodiments, a gasket 7 is disposed between the light cylinder 2 and the heating sheet 3. The gasket 7 functions to separate the heating sheet 3 from an outer wall of the light cylinder 2, so as to prevent the heat generated by the heating sheet 3 from entering the interior of the light cylinder 2 to affect normal operation of the power supply 6, the PCB 1, or other elements.

In order to prevent displacement of the gasket 7, a heating seat 8 is disposed on an outer surface of the light cylinder 2, and the heating seat 8 is adapted to the gasket 7. When the gasket 7 is assembled, the gasket 7 is inserted into the heating seat 8 of the light cylinder 2 to facilitate assembly. The heating seat 8 is made of an acrylonitrile butadiene styrene (ABS) material, and the heating seat 8 separates the heating sheet 3 from the light body of the light cylinder 2 to greatly reduce heat propagation to the light body of the light cylinder 2. The gasket 7 enables the heating sheet 3 to tightly adhere to the sleeve 4, resulting in faster heat transfer to the sleeve and faster heating effect.

Preferably, the gasket 7 in the foregoing embodiment is made of EPDM rubber, and the sleeve 4 is made of aluminum. The gasket 7 made of EPDM rubber has strong heat resistance and can be used for a long term in high-temperature environments. The aluminum has good thermal conductivity, which can improve thermal conductivity efficiency of the heating sheet 3 inside the light.

For safety reasons, the PCB 1 in the foregoing embodiment is electrically connected with a thermistor, which is placed on the gasket 7 and wrapped by the heating sheet 3. When the heating sheet 3 overheats, a resistance value of the thermistor increases, and current input from the PCB 1 to the heating sheet 3 decreases, whereby the heat provided by the heating sheet 3 will not scald hands.

Described above is only an embodiment of the present utility model, and a specific structure and characteristics commonly known in the solution are not described in detail here. It should be pointed out that, for those skilled in the art, several modifications and improvements may be made without departing from the structure of the present utility model, and these modifications and improvements should also be considered as the protection scope of the present utility model and will not affect the effects of implementation of the present utility model and the practicality of the patent. The protection scope of the present application shall be subject to the content of the claims, and the specific embodiments and other descriptions in the specification may be used for explaining the content of the claims.

## Claims

1. A light with a heating function, comprising a PCB (1), a light cylinder (2), a heating sheet (3), and a sleeve (4), wherein the heating sheet (3) is electrically connected to the PCB (1), the sleeve (4) is sheathed outside the light cylinder (2), and the heating sheet (3) is disposed between the light cylinder (2) and the sleeve (4); and comprising a heating switch (5) and a power supply (6), wherein the power supply (6) and the heating switch (5) are electrically connected to the PCB (1) separately, and the power supply (6) and the PCB (1) are mounted inside the light cylinder (2).

2. The light with a heating function according to claim 1, wherein a gasket (7) is disposed between the light cylinder (2) and the heating sheet (3).

3. The light with a heating function according to claim 2, wherein a heating seat (8) is disposed on an outer surface of the light cylinder (2), and the heating seat (8) is adapted to the gasket (7).

4. The light with a heating function according to claim 2, wherein the gasket (7) is made of EPDM rubber.

5. The light with a heating function according to claim 1, wherein the sleeve (4) is made of aluminum.

6. The light with a heating function according to claim 3, wherein the PCB (1) is electrically connected with a thermistor, which is placed on the gasket (7).
